# EUROPEAN PATENT SPECIFICATION

(11) **EP 2 072 032 B1**
(45) Date of publication and mention of the grant of the patent: **20.06.2012**
(21) Application number: 08103332.6
(22) Date of filing: 02.04.2008
(51) Int. Cl.: A61K 8/14, A61K 8/60, A61Q 19/08

(54) **Method for the intracellular regeneration of hyaluronic acid and cosmetic composition therefor**
Verfahren für die intrazelluläre Regeneration von Hyaluronsäure und dazu gehörende kosmetische Zusammensetzung
Procédé de régénération intracellulaire d'acide hyaluronique et composition cosmétique associée

(30) Priority: 17.12.2007 IT MI20072348
(43) Date of publication of application: 24.06.2009
(73) Proprietor: Medichem S.r.L., 20063 Cernusco Sul Naviglio (MI) (IT)
(72) Inventor: Cairo, Marcello, 20063, CERNUSCO SUL NAVIGLIO (MI) (IT); Perugini, Paola, 27100, PAVIA (IT)
(74) Representative: Banfi, Paolo

(56) References cited:
- EP-A- 0 295 092
- WO-A-95/24497
- US-A1- 2007 077 292

## Description

The present invention relates to a cosmetic method for the regeneration of hyaluronic acid in cells of connective tissues, starting from the hyaluronic acid precursors D-glucuronic acid and N-acetyl-D-glucosamine, or derivatives thereof. More specifically the invention provides a cosmetic composition containing liposomes loaded with D-glucuronic acid and N-acetyl-D-glucosamine, or derivatives thereof, in admixture with a physiologically-acceptable carrier.

### Background of the invention

The use of liposomes for the delivery of hyaluronic acid is described in several patent documents. To cite a few examples, WO2006122638 discloses a filler for use in dermocosmetics, containing hyaluronic acid or derivatives thereof structured in phospholipid liposomes. WO03000190 and WO03000191 disclose a method for intra-articular delivery of hyaluronic acid encapsulated in liposomes for the treatment of osteoarthritis. WO9813024 discloses a pharmaceutical formulation containing a mixture of hyaluronic acid and liposomes.

EP-A- 295 092 discloses cosmetic preparations containing liposome vesicles loaded with hyaluronic acid fragments.

US2007/077292 discloses cosmetic preparations containing liposome vesicles loaded with hyaluronic acid.

### Description of the invention

The present invention is based on the finding of an effective method for regenerating hyaluronic acid inside the cells by administering, to a subject in need thereof, a liposomal preparation containing separately - i.e. not linked by chemical bond - the hyaluronic acid precursors D-glucuronic acid (GA) and N-acetyl-D-glucosamine (GlcNH2), or derivatives thereof. The regeneration of hyaluronic acid inside the cells enables rebuilding the connective tissue damaged by age-related degenerative processes. Such a regenerative effect is particularly useful in dermatology, especially for the treatment of wrinckles and skin-aging.

Accordingly, in a first embodiment the invention provides a cosmetic preparation containing liposomal vesicles loaded with D-glucuronic acid and N-acetyl-D-glucosamine, or derivatives thereof, in admixture with a cosmetically acceptable carrier. As used herein, the term "derivative" indicates an activated form of D-glucuronic acid and N-acetyl-D-glucosamine, the uridindiphospho-derivatives UDP-D-glucuronic acid and UDP-N-acetyl-D-glucosamine. The two active ingredients (GA and GlcNH2 or their derivatives) can be loaded in the same liposomal vesicles or a mixture of vesicles encapsulating either one or the other active ingredient can be used in accordance with the invention. The composition may also contain other ingredients that facilitate the regeneration of hyaluronic acid inside the cells, e.g. by inhibiting its degradation by hyaluronidase. Preferably the hyaluronidase inhibitor is an extract of echinacea angustifolia.

The liposomal vesicles may be unilamellar (SUV) or multilamellar (MLV) vesicles of various size (small, medium, large, giant), and may be formed by phospholipids or sphingolipids, preferably by phosphatidylcholine; the vesicles can also contain ingredients modulating their resistance, viscosity or stability.

Methods known in the art can be used to load liposome vesicles with D-glucuronic acid and/or N-acetyl-D-glucosamine or derivatives thereof; for example, the phospholipids may be added to a mixture of the two components, or separately to each single component dissolved in a suitable medium, under constant stirring, followed by turbine homogenization. In cases where the active components are separately added to liposomal vesicles, these are subsequently mixed to form a liposomal mixture of both active ingredients. Once the process is terminated, the liposomes can be dispersed in a cosmetic base or preparation, to form a cream, emulsion, microemulsion, ointment, gel, lotion and oil.

In a preferred embodiment, the liposomal preparation is dispersed in a cosmetic base containing water and glycerol, prefarably in the presence of additional ingredients such as EDTA, sodium pyroglutamate, sodium lactate, xanthan gum or similar thickening agents, under conditions suitable for forming a gel paste.

In a further embodiment the invention provides the use of the compositions herein disclosed for dermatological treatments, particularly for the treatment of unaesthetisms such as wrinkles, skin folds, acne scars, post-injury scars, rhinoplasty scars, lips or face (checks and chin) lift.

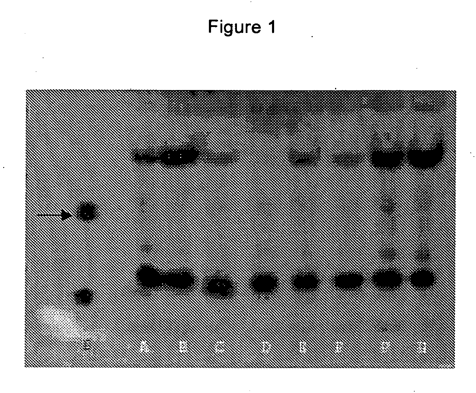

### Description of Figure 1

PAGEFS electrophoresis of cell cultures; the arrow indicates the disaccharide band corresponding to hyaluronic acid;
S: disaccharide solution indicative of hyaluronic acid;
A-B: control plates;
C: plate treated with placebo liposomes 15 mg/ml;
D: plate treated with placebo liposomes 60 mg/ml;
E-F: plates treated with liposomes loaded with GA and GlcNH2 at 0.5 and 10 mM concentrations, respectively;
G-H: plates treated with liposomes loaded with GA and GlcNH2 at 5 e 50 mM concentrations, respectively.

### Example 1 - Preparation of phosphatidylcholine-based liposomal systems for the delivery of D-glucuronic acid (GA) and N-acetyl-D-glucosamine (GlcNH2) and analysis of the content of active ingredients

### Materials

Phosphatidylcholine used: PhosPho LCN-DS, soy lecithin, minimum 62% purity, Fancor; Egg phosphatidylcholine (PC), XI-E type, 99% purity, Sigma.

D-glucuronic acid sodium salt monohydrate, N-acetyl-L-glucosamine, Aldrich; Cholesterol, Carlo Erba.

Liposomes were prepared with two methods to increase the encapsulation of GA and GlcNH2.

The following variables were introduced:
- preparation method (direct hydration or hydration after forming a lipidic film)
- quantity and type of phosphatidylcholine
- use of cholesterol
- concentrations of GA and GlcNH2 solutions

### METHOD A (direct hydration):

The following procedure was followed for 'placebo' and 'loaded' batches:
- weigh the lipophilic phase and add the hydrophilic phase (final volume from 200 µl to 1 ml);
- pass in a vortex until complete phospholipid dispersion and subsequently in termomixer for 15 min at 25°C, 1400 rpm;
- in certain cases maintain the dispersion at 4°C for 24 hrs;
- centrifuge the samples for 60 min at 4°C, 16400 rpm to eliminate the active ingredient that was not encapsulated;
- separate the surnatant from the pellet and maintain the two parts separately freezed at -20°C for subsequent specific dosage of the active ingredients.

By this method, batches formed by phosphatidylcholine PhosPho LCN-DS were produced; the composition of these batches is reported in Table 1. Unless otherwise specified, the hydrophilic phase volume was maintained at 1 ml. Batches marked with * were hydrated for 24 hrs in a refrigerator prior to centrifugation.

**Table 1**

| Batch | Phosphatidylcholine amount | Hydrophilic phase | Active ingredient concentration (µmol/ml) |
|---|---|---|---|
| JALU 3.7 | 70 mg | Water | - |
| JALU 20-22 | 60 mg | Water | - |
| JALU 35 | 600 mg | Water (10 ml) | - |
| JALU 42 | 15 mg | Water (200 µl) | - |
| JALU 14-16 | 15 mg | Water | - |
| JALU 8-10 | 7 mg | Water | - |
| JALU 4-6 | 70 mg | GA 1.5 mg | 6.4 |
| JALU 23-25 | 60 mg | GA 0.3 mg | 1.3 |
| JALU 17-19 | 15 mg | GA 0.3 mg | 1.3 |
| JALU 11-13 | 7 mg | GA 0.3 mg | 1.3 |
| JALU 36-38 | 15 mg | GA 1 mg | 4.3 |
| JALU 39-41 | 15 mg | GA 1 mg (200 µl) | 21.5 |
| JALU 56-58 | 15 mg | GA 5 mg (200 µl) | 105 |
| JALU 63 | 75 mg | GA 25 mg | 105 |
| JALU 32-34 | 60 mg | GlcNH2 0.3 mg | 1.35 |
| JALU 29-31 | 15 mg | GlcNH2 0.3 mg | 1.35 |
| JALU 26-28 | 7 mg | GlcNH2 0.3 mg | 1.35 |
| JALU 50-52 | 15 mg | GlcNH2 5 mg | 22.6 |
| JALU 59-61 | 15 mg | GlcNH2 5 mg (200 µl) | 113 |
| JALU 64-66* | 15 mg | GA 5 mg (200 µl) | 105 |
| JALU 67-69* | 30 mg | GA 5 mg (200 µl) | 105 |
| JALU 71-73* | 15 mg | GlcNH2 5 mg (200 µl) | 113 |
| JALU 74-76* | 30 mg | GlcNH2 5 mg (200 µl) | 113 |
| JALU 77-79* | 15 mg | GlcNH2 10 mg (400 µl) | 226 |

### METHOD B

Liposome batches were produced using both phosphatidycholine PhosPho LCN-DS and PC. Also in this case 'placebo' and 'loaded' batches were produced, according to the following procedure:
- weigh phosphatidylcholine in a round-bottomed flask
- add 500 µl CHCl3 containing 10 mg/ml cholesterol
- dry in rotavapor under reduced pressure at 40°C for 10 min
- rehydrate with 1 ml hydrophilic phase
- vortex for 10 min, centrifuge for 60 min at 4°C, 16400 rpm
- separate surnatant and pellet and freeze at -20°C.

### Table 2 reports the liposome batches produced with method B

**Table 2**

| Batches | Phosphatidylcholine (mg) | Hydrophilic phase | Active ingredient concentration (µmol/ml) |
|---|---|---|---|
| JALU 43-45 | PhosPho LCNDS (15 mg) | GA 1 mg | 4.3 |
| JALU 46-48 | PC (10 mg) GA | 1 mg | 4.3 |
| JALU 49 | PC (10 mg) | Water | - |
| JALU 62 | PhosPho LCNDS (15 mg) | Water | - |
| JALU 53-55 | PhosPho LCNDS 15 mg | GA 5 mg | 21 |

### Size determination

A dimensional analysis of the liposome systems was carried out using the laser diffraction apparatus Mastersizer 2000 (Malvern), which operates in a size interval ranging from 20nm to 2mm. The apparatus is equipped with a dispersion unit connected to a pump to shake the sample during the analysis. Each sample was analysed in triplicate and the results are expressed as d₁₀, d₅₀ and d₉₀, i.e. the diameter of 10%, 50% and 90% of the analysed sample.

Table 3 reports the analysis carried out on placebo batches at different concentrations of phosphatidylcholine, prepared according to each method.

**Table 3**

| Batch | Amount of phosphatidylcholine | D_{10%} (µm) | D_{50%} (µm) | D_{90%} (µm) |
|---|---|---|---|---|
| JALU 8-10 | PhosPho LCNDS 7 mg | 1.199 | 5.443 | 52.251 |
| JALU 14-16 | PhosPho LCNDS 15 mg | 1.598 | 8.766 | 52.908 |
| JALU 20-22 | PhosPho LCNDS 60 mg | 1.612 | 26.459 | 102.922 |
| JALU 49** | PC (10 mg) | 2.639 | 6.359 | 14.602 |
| JALU 62** | PhosPho LCNDS (15 mg) | 2.903 | 14.162 | 47.823 |

| | | | | |
|---|---|---|---|---|
| ** indicate the batches prepared with method B | | | | |

The results evidence the formation of vesicles of micrometric size; as far as PhosPho LCNDS is concerned, the size is constant using either of the two methods with the same amount of phospholipids; the results of sample Jalu 49 indicate that the size depends on the purity of the starting phospholipid.

### Example 2 - gel preparation

### Phase A (Liposome formation)

The following components are exactly weighed and placed in a inox-steel reactor equipped with a variable-speed turbine (0-3000 rounds/min) and with a paddle agitator, in the order:
- DEMINERALIZED WATER 56.400%
- GA 0.100%
- GlcNH2 1.100%
the components are mixed until a complete dissolution is obtained.
Phase A' is added slowly under turbine (2.500 rounds/min):
- LECITHIN 1.500%
homogenization is carried out for 15 min. The turbine is stopped and mixing is continued with the paddle agitator for additional 10 min.

### Phase B

In a separate steel reactor equipped with heating, stirring and turbine, the following solution is prepared:
- DEMINERALIZED WATER 30.000%
- DISODIUM EDTA 0.500%
- GLYCEROL 8.000%
- SODIUM PYROGLUTAMATE 1.000%
- SODIUM LACTATE 1.000%
the solution is shaken until it becomes homogeneous and the mass is heated at 70°C, then Phase B' is added slowly under the turbine (1.500 rounds/min):
- XANTHAN GUM 0.400%
homogenization is carried out until complete dispersion of xanthan gum with formation of a compact and homogeneous gel. The mixture is cooled to 25°C and the Phase A is poured under stirring. Stirring is continued for additional 20 min until a homogeneous and compact mass is obtained.

### Example 3 - gel preparation (alternative 1)

### Phase A (1^{st} liposome)

The following components are exactly weighed and placed in a inox-steel reactor equipped with a variable-speed turbine (0-3000 rounds/min) and with a paddle agitator, in the order:
- DEMINERALIZED WATER 18.800%
- GLUCURONIC ACID 0.100%
the components are mixed up to complete dissolution. LECITHIN (0.500%) is added slowly under turbine (2.500 rounds/min). Homogenization is continued for 15 min. The turbine is stopped and mixing is continued with the paddle agitator for additional 10 min.

### Phase A' (2^{nd} liposome)

The following components are exactly weighed and placed in a inox-steel reactor equipped with a variable-speed turbine (0-3000 rounds/min) and with a paddle agitator, in the order:
- DEMINERALIZED WATER 18.800%
- N-ACETYL-L-GLUCOSAMINE 1.100%
the components are mixed up to complete dissolution. LECITHIN (0.500%) is added slowly under turbine (2.500 rounds/min). Homogenization is continued for 15 min. The turbine is stopped and mixing is continued with the paddle agitator for additional 10 min.

### Phase A" (3^{rd} liposome)

The following components are exactly weighed and placed in a inox-steel reactor equipped with a variable-speed turbine (0-3000 rounds/min) and with a paddle agitator, in the order:
- DEMINERALIZED WATER 18.800%
- Echinacea angustifolia (dry extract) 0.200%
the components are mixed up to complete dissolution. LECITHIN (0.500%) is added slowly under turbine (2.500 rounds/min). Homogenization is continued for 15 min. The turbine is stopped and mixing is continued with the paddle agitator for additional 10 min.

### Liposome preparation

In an inox-steel reactor equipped with agitator, the three phases A, A' and A" are mixed slowly for 10 min until complete homogenization is obtained.

### Phase B

In an inox-steel reactor equipped with heating, stirring and turbine the following solution is prepared:
- DEMINERALIZED WATER 29.800%
- DISODIUM EDTA 0.500%
- GLYCEROL 8.000%
- SODIUM PYROGLUTAMATE 1.000 %
- SODIUM LACTATE 1.000%

The mixture is stirred up to homogeneity and heated at 70°C. Once the temperature is reached, Phase B' is added slowly under turbine (1.500 rounds/min):
- XANTHAN GUM 0.400%

Homogenization is continued until the dispersion of Xanthan gum is complete and a homogeneous and compact gel is obtained. The temperature is cooled to 25°C and the mass is added to the liposome preparation under stirring. Stirring is continued for 20 min until a homogeneous and compact mass is obtained.

### Example 4 - gel preparation (alternative 2)

### Phase A (1^{st} liposome)

The following components are exactly weighed and placed in a inox-steel reactor equipped with a variable-speed turbine (0-3000 rounds/min) and with a paddle agitator, in the order:
- DEMINERALIZED WATER 18.800%
- UDP - D-GLUCURONIC ACID 0.100%
the components are mixed up to complete dissolution. LECITHIN (0.500%) is added slowly under turbine (2.500 rounds/min). Homogenization is carried out for 15 min. The turbine is stopped and mixing is continued with the paddle agitator for additional 10 min.

### Phase A' (2^{nd} liposome)

The following components are exactly weighed and placed in a inox-steel reactor equipped with a variable-speed turbine (0-3000 rounds/min) and with a paddle agitator, in the order:
- DEMINERALIZED WATER 18.800%
- UDP-N-ACETYL-L-GLUCOSAMINE 1.100%
the components are mixed up to complete dissolution. LECITHIN (0.500%) is added slowly under turbine (2.500 rounds/min). Homogenization is continued for 15 min. The turbine is stopped and mixing is continued with the paddle agitator for additional 10 min.

### Phase A" (3^{rd} liposome)

The following components are exactly weighed and placed in a inox-steel reactor equipped with a variable-speed turbine (0-3000 rounds/min) and with a paddle agitator, in the order:
- DEMINERALIZED WATER 18.800%
- Echinacea angustifolia (dry extract) 0.200%
the components are mixed up to complete dissolution. LECITHIN (0.500%) is added slowly under turbine (2.500 rounds/min). Homogenization is continued for 15 min. The turbine is stopped and mixing is continued with the paddle agitator for additional 10 min.

### Liposome preparation

In an inox-steel reactor equipped with agitator, the three phases A, A' and A" are mixed slowly for 10 min until complete homogeneity is obtained.

### Phase B

In an inox-steel reactor equipped with heating, stirring and turbine the following solution is prepared:
- DEMINERALIZED WATER 29.800%
- DISODIUM EDTA 0.500%
- GLYCEROL 8.000%
- SODIUM PYROGLUTAMATE 1.000%
- SODIUM LACTATE 1.000%

The mixture is stirred up to homogeneity and heated at 70°C. Once the temperature is reached, Phase B' is added slowly under turbine (1.500 rounds/min):
- XANTHAN GUM 0. 400%

Homogenization is continued until the dispersion of Xanthan gum is complete and a homogeneous and compact gel is obtained. The temperature is cooled to 25°C and the mass is added to the liposome preparation under stirring. Stirring is continued for 20 min until a homogeneous and compact mass is obtained.

### Example 5 - set up of a cellular system for the in vitro evaluation of the liposome systems

The studies on the cellular effects were carried out with NHDF cells (Normal Human Dermal Fibroblasts), 6 passages in DMEM, 10% FBS, 1% PenStrep, 1% glutamine, in multiwell plates (growth area for single well = 9.6 cm²). Each well contained 300,000 confluent cells.

Samples were inoculated according to the following scheme, in sterile conditions and under laminar-flow at 37°C placing 2 ml medium in each well and the wells were incubated at 37°C for 60 hrs, 5% CO2, 97% humidity.

Scheme of the samples inoculated in 8 plates:
➢ A-B control plate;
➢ C plate treated with liposomes (control) 15 mg/ml;
➢ D plate treated with liposomes (control) 60 mg/ml;
➢ E-F plates treated with liposomes loaded with GA and GlcNH2 at 0.5 and 10 mM concentrations, respectively.
➢ G-H plates treated with liposomes loaded with GA and GlcNH2 at 5 and 50 mM concentrations, respectively.

At the end of the scheduled time period, plates were analysed with phase-contrast optical microscopy, whereby cell death was found in plates D, E, F, G, H. Surnatants were recovered, centrifuged and examined for their hyaluronic acid content. In brief, 1 ml clarified medium was liophilized, taken up with 300 ml ammonium acetate pH 7.0 and precipitated with 4 volumes of cold ethanol and left at -20°C for 16-18 hrs, centrifuged and the pellet was recovered; this step was repeated a second time. At the end of the second precipitation, the pellet was completely dried and digested with 100 mU/ml hyaluronidase SD at 37°C for 1 h and with 100 mU/ml Condroitinase ABC at 37°C for 3 h. The samples were then liophilized and derivatized with 2-aminoacridone (AMAC).

40 µl aliquots of 12.5 mM AMAC solution in acetic acid/DMSO (3:17, v/v) were added to each sample and incubated for 10-15 min at room temperature; then 40 µl of 1.25 M NaBH3CN fresh solution were added and the samples incubated for 16-18 hrs at 37°C. After the derivatization, the samples were separated by PAGEFS.

Figure 1 shows that the disaccharide band, which is indicative of hyaluronic acid synthesis, is present in samples containing liposomes and results particularly thicker, which means that a higher concentration of hyaluronic acid is formed, in the samples containing a higher concentration of GA and GlcNH2 precursors.

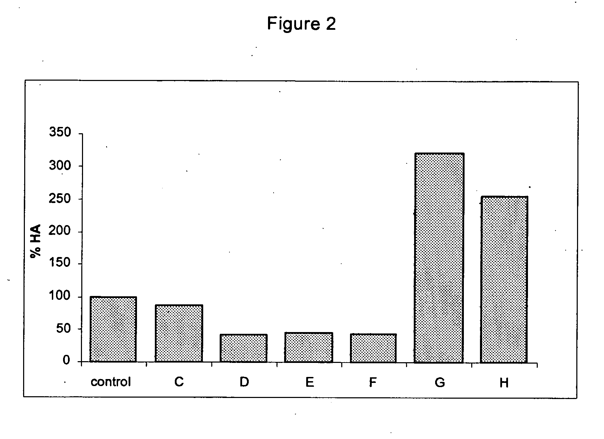

In Figure 2 the results are expressed as % hyaluronic acid production with respect to the control, assumed 100%.

### Conclusions

The incubation time in this experiment (60 hrs) resulted too long for cell survival.

The electrophoresis shows that the samples treated with phospholipid concentrations of 15 and 60 mg/ml do not present inhibitory effect on the biosynthesis of hyaluronic acid.

The inoculum of liposomes containing GA and GlcNH2 at 5 and 50 mM concentrations, respectively, induce an increase of the disaccharide band which is indicative of hyaluronic acid synthesis.

### Example 6 - In vitro efficacy study

Based on the previous results, the following method was carried out: NHDF (Normal Human Dermal Fibroblasts) cells, 6 passages in DMEM, 10% FBS, 1% PenStrept, 1% glutamine, in multiwell 6 plates (growth area for each single well = 9.6 cm2). Each well was loaded with 300.000 confluent cells.

Samples were inoculated in sterile conditions, at 37°C with 2 ml medium added to each well and incubated at 37° for 48 or 72 hours, 5% CO2, 97% humidity. 12 hrs after the inoculum, fresh medium was added.

Samples inoculated in 12 plates:
- 2 control plates 48hr;
- B-C plates treated for 48 hrs with liposomes loaded with GA and GlcNH2 at 0.25 and 5 mM concentrations, respectively;
- H-I plates treated for 48 hrs with liposomes loaded with GA and GlcNH2 at 0.5 and 10 mM concentrations, respectively;
- 2 control plates 72 hrs;
- D-E plates treated for 72 hrs with liposomes loaded with GA and GlcNH2 at 0.25 and 5 mM concentrations, respectively;
- F-G plates treated for 72 hrs with liposomes loaded with GA and GlcNH2 at 0.5 and 10 mM concentrations, respectively.

At the end of the scheduled time period, plates were analysed with phase-contrast optical microscopy and living cells were counted.

The analysis of hyaluronic acid produced by cells is carried out on the medium treated with specific enzymes (hyaluronidase and condroitinase) by means of a chromatographic analysis specific for proteins (FPLC). The separation is carried out on a pre-packed column Superdex 200HR 10/30 at room temperature, with a flow rate of 0.35 ml/min; ammonium acetate 0.1M pH7, containing 0.05% Tween 20 was used as the buffer. The UV-Vis detector was set at 280 nm.

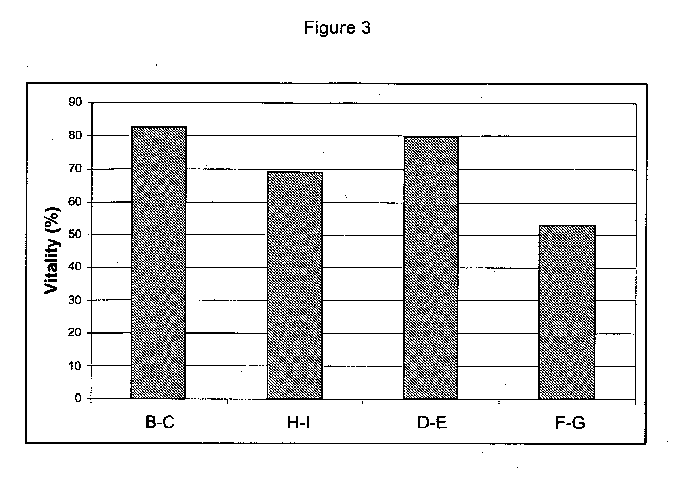

The results for cell viability, expressed as percentage of vital cells compared to controls, are reported in Figure 3. Particularly positive results were obtained with plates B-C and D-E wherein viability resulted higher than 80%.

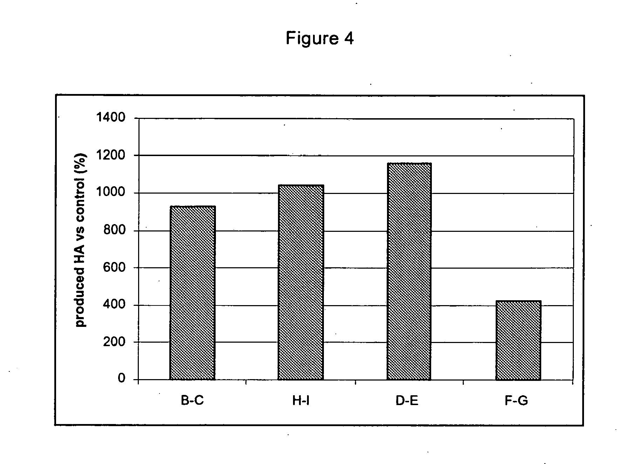

The results in terms of production of hyaluronic acid at 48 and 72 hrs are reported in Figure 4. The results are expressed as HA% determined as the ratio of the amount of HA found in cells treated with liposomes to the amount produced by control cells.

The results demonstrate that the production of hyaluronic acid is by far higher in treated plates than in controls; this indicates that the liposomal systems effectively deliver the two hyaluronic acid precursors into fibroblasts, leading to the (re)constitution of this essential component of derma structure and skin.

### Example 7 - in vivo efficacy study using non-invasive methods for cutaneous bioengineering

Population: 20 healthy volunteers (males and females) were selected on the basis of the following general exclusion criteria:
individuals presenting, in the investigated cutaneous area, signs that could interfere with the assay results; hypersensitivity or intolerance known for cosmetic products and/or products for body cleaning/detersion, including sunscreens; sun-exposure susceptibility; subjects treated with photosensitive drugs less than one month earlier; individuals treated with UVA and/or UVB radiations less than one month earlier.

### Tested formulations

Formulation A: glucuronic acid 0.1%, acetyl glucosamine 1.1%, phospho LCN-DS 1.5%, EDTA 2Na 0.5%, glycerol 8%, PCA Na 1%, Na lactate 1%, Sepiplus 400 2.3%, demineralized water q.s. to 100.

Formulation B, used as placebo: phospho LCN-DS 1.5%, EDTA 2Na 0.5%, glycerol 8%, PCA Na 1%, Na lactate 1%, Sepiplus 400 2.3%, demineralized water q.s. to 100.

### Anatomic area of application

The subjects, divided in groups A and B, were requested to apply the formulation A or B, respectively, to the left forearm, whereas right forearms were used as controls.

### Dose and administration

The formulations were applied once a day
Treatment period: 2 months
Biometric evaluation

The following parameters were considered in this study: water content of horny layer, cutaneous firmness (R0), total (R2) and neat elasticity (R5), erythema degree, skin-feeling. The measurement of cutaneous parameters was carried out at the beginning of the experiment (t0) and after 15 (t1), 30 (t2) and 60 (t3) days by means of the multi-probe Cutometer® MPA 580 (CK electronic GmbH, G.F. Secchi, CO), according to the international guidelines.

### Simultaneous application of different products

The subjects were adviced not to apply other cosmetic products to the treated areas, 3 days in advance and during the period of study, to avoid uncertain results.

### Results

### Erythema index

In order to ascertain the tolerability of the applied formulations on skin, the erythema level on the treated and control sites was evaluated, for all the time periods checked during the study. It is known that an increase of the erythema level following the application of a formulation indicates scarce tolerability of the formulation itself.

The results obtained in this study are herein reported as erythema indexes, i.e. as ratio between the erythema level at time t and the basic erythema level for every site examined.

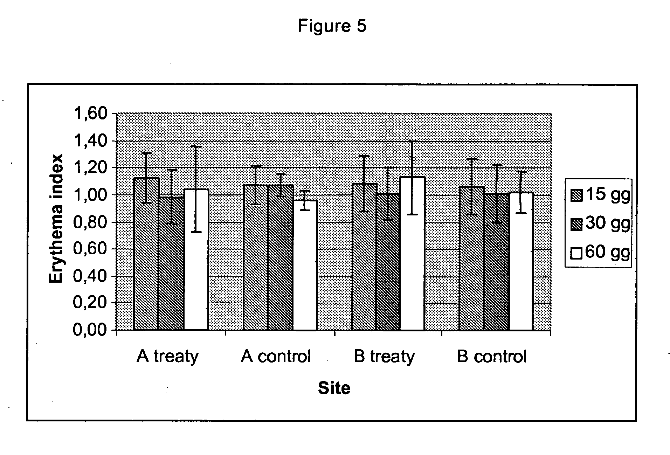

When comparing the histogram referred to the application site with the control (Figure 5) for each time period, no significant differences may be noted; therefore, no increase of the erythema level may be associated to the application of the two formulations.

Further, when observing the graphs obtained for each formulation in time, it is noted that the erythema indexes keep close to unit for all times taken into consideration: the application of the two formulations does not cause a change of the erythema level even after a prolonged application.

Therefore, both the formulations used in this study are well tolerated and no irritation can be ascribed to the vehicle, to liposomes or to the active ingredients at the studied concentrations.

### Skin hydration

The table 4 below reports the average hydration values of the population under exam obtained with a corneometer as well as the change with respect to the forearm used as control at each time.

**Table 4**

| Formulations | | T_{15days} | T_{30days} | T_{60days} |
|---|---|---|---|---|
| Formulation A | average ± s.d. | 43.74±7.62 | 47.52±9.70 | 44.09±7.10 |
| | % vs. | (+34.05) | (+38.18) | (+28.96) |
| | control | | | |
| Formulation B | average ± s.d. | 46.47±7.4 | 43.18±5.15 | 44.25±6.94 |
| | % vs. | (+35.88) | (+22.88) | (+24.08) |
| | control | | | |

By comparing the corneometry values from forearms treated with the corresponding controls, it is noted that the significant increase appears very similar after 15 days application (34.05% and 35.88%). From the above data, it is evident that the application of both formulations is accompanied by an increase of water content in the corneal layer, wherein such hydration improvement can be ascribed to the composition of the formulations and to the presence of liposomes, regardless of the active ingredients contained therein.

Following repeated applications of formulation A for 30 days, an increase is obtained 66.87% higher than the increase due to the application of formulation B. The greater increase of surface hydration after repeated application of formulation A is probably due to the presence of active ingredients inside liposomes.

Such hydration increase in subjects belonging to group A is not maintained as such after 60 days daily application (28.96% vs. 24.08% of group B). This result may be due to a stability problem of the active ingredients contained in the liposomal structures.

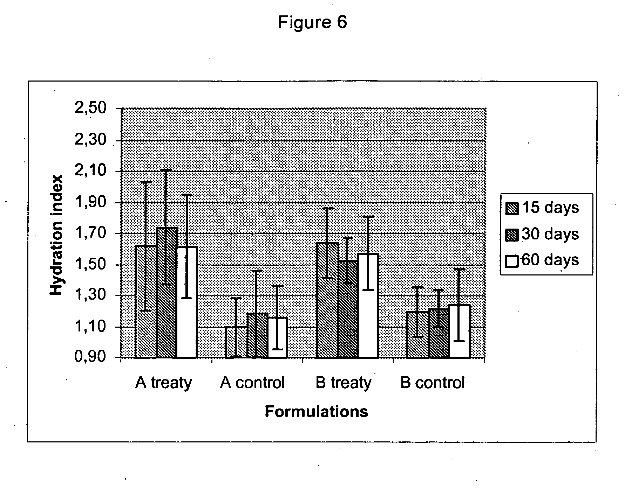

The study results are reported in Figure 6 as hydration indexes, i.e. as ratio between the values obtained at different times t and the basal values (TO).

Even by comparing the results with the basal values, skin hydration increase following application of the formulations may be observed at all times evaluated in this study.

### Skin elasticity

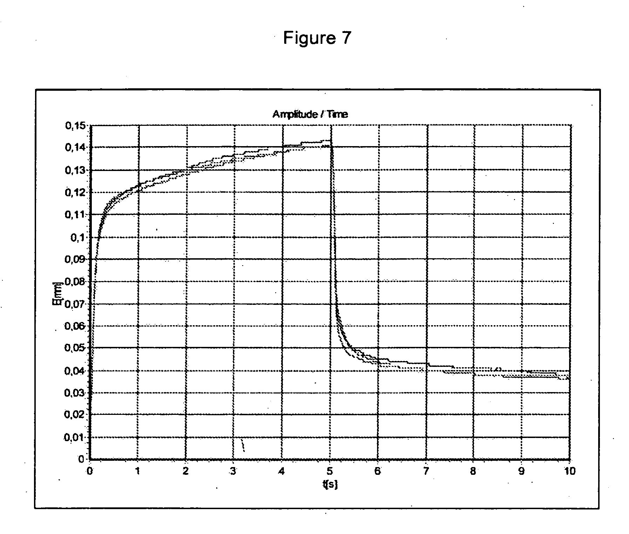

Skin elasticity and firmness change after application of the formulations is assayed by means of a software programmed to carry out a number of operations with curves obtained after measuring the analyzed area. Exemplary curves obtained during the study are reported in Figure 7.

The following, internationally-approved parameters for the evaluation of elasticity and firmness, may be obtained from such curves:
R0 = highest point of the first curve; skin firmness index;
R2 = curve portion comprised between the maximum width and the skin ability to re-deform. This parameter is very important since it indicates the total skin elasticity.
R5 = This parameter indicates the actual elasticity, that is the total elasticity minus the skin viscoelastic portion.

The results relating to this three parameters will be discussed separately.

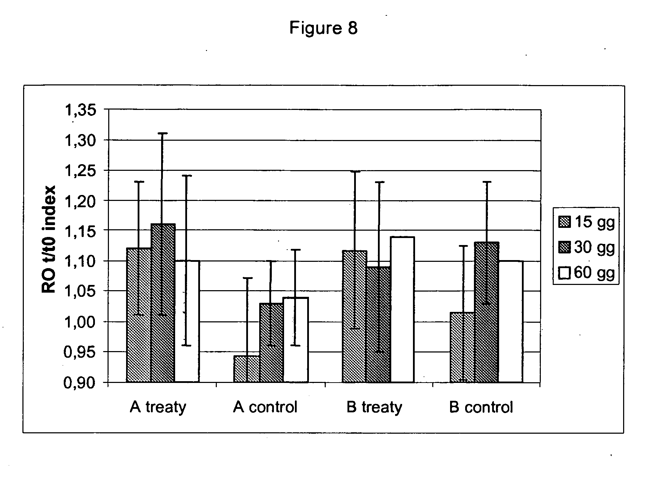

As to skin firmness, Figure 8 reports the variation of the R0 index with respect to the starting value, for both tested formulations, in treated and control areas.

The results show that after 15 days treatment, both formulations have a definitively higher index with respect to the non-treated areas, showing a clear improvement in skin firmness; after 30 or more days treatment, the improvement is evident only for formulation A, which contains the active ingredients.

These results are perfectly consistent with the results relating to skin hydration.

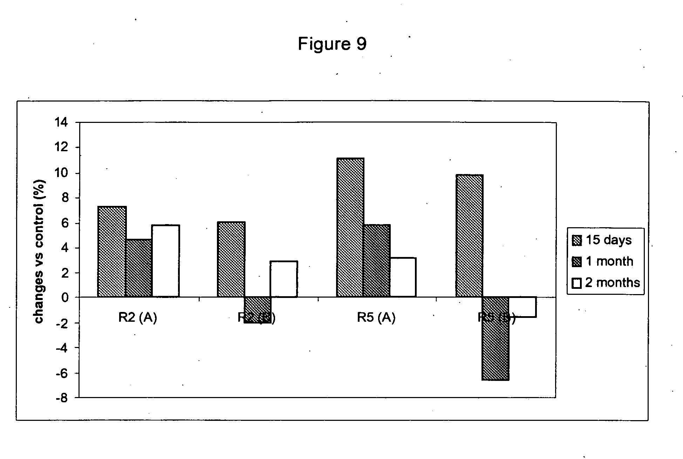

As to skin elasticity parameters, Figure 9 shows the percent variation of indexes R2 and R5 compared to the non-treated area, for both the tested formulations.

The results are in line with those reported above: after 15 days treatment, a positive effect due to the application of formulations containing liposomes may be observed whereas, in case the application is prolonged, the specific action of glucuronic acid and of glucosamine contained in liposome systems may result in a consistent increase of skin elasticity.

### CONCLUSIONS

1. lyposomes containing glucuronic acid and acetyl-glucosamine have suitable characteristics of size and active-ingredients content.
2. the in vitro results clearly show an increase in the amount of hyaluronic acid produced by cells after incubation of the precursor-containing liposomes, thus pointing out the ability of liposomal systems to deliver active ingredients inside fibroblasts.
3. the formulation containing glucuronic acid and acetyl-glucosamine proved well-tolerated after a continued two-month use; such formulation proved also capable to remarkably improve skin hydration, firmness and elasticity parameters after 30 days treatment with respect to the formulation containing placebo liposomes.

## Claims

1. A cosmetic preparation containing liposome vesicles loaded with
i) D-glucuronic acid or UDP-D-glucuronic acid, and
ii) N-acetyl-D-glucosamine or UDP-N-acetyl-D-glucosamine, in admixture with a cosmetically-acceptable carrier.

2. A preparation according to claim 1, further containing a hyaluronidase inhibitor.

3. A preparation according to claim 2, wherein said hyaluronidase inhibitor is an extract of *echinacea angustifolia.*

4. A preparation according to claims 1-3, wherein the vesicles consist essentially of phosphatidylcholine.

5. A preparation according to claims 1-4, which is in the form of cream, emulsion, microemulsion, ointment, gel, lotion or oil.

6. A preparation according to claim 5, which is in the form of a gel containing water and glycerol.

7. A preparation according to claim 6, further containing EDTA, sodium pyroglutamate, sodium lactate and a thickening agent, preferably xanthan gum.

8. The use of liposomes encapsulating
i) D-glucuronic acid or UDP-D-glucuronic acid, and
ii) N-acetyl-D-glucosamine or UDP-N-acetyl-D-glucosamine, in a cosmetic composition.

9. The use of a preparation according to claims 1-7, for the non-therapeutic cosmetic treatment of wrinkles, skin folds, acne scars, post-injury scars, rhinoplasty scars, lifted, lips cheeks or chin.

## Patentansprüche

1. Kosmetisches Präparat enthaltend Liposomvesikel, welche beladen sind mit
i) D-Glucuronsäure oder UDP-D-Glucuronsäure, und
ii) N-Acetyl-D-Glucosamin oder UDP-N-Acetyl-D-Glucosamin, im Gemisch mit einem kosmetisch verträglichen Träger.

2. Präparat gemäß Anspruch 1, des Weiteren enthaltend einen Hyaluronidaseinhibitor.

3. Präparat gemäß Anspruch 2, wobei der Hyaluronidaseinhibitor ein Extrakt aus *Echinacea angustifolia* ist.

4. Präparat gemäß den Ansprüchen 1 bis 3, wobei die Vesikel im Wesentlichen aus Phosphatidylcholin bestehen.

5. Präparat gemäß den Ansprüchen 1 bis 4, welches in Form einer/eines Creme, Emulsion, Mikroemulsion, Salbe, Gels, Lotion oder Öls ist.

6. Präparat gemäß Anspruch 5, welches in Form eines Wasser und Glycerol enthaltenden Gels ist.

7. Präparat gemäß Anspruch 6, des Weiteren enthaltend EDTA, Natriumpyroglutamat, Natriumlactat und ein Verdickungsmittel, vorzugsweise Xanthan.

8. Verwendung von Liposomen, die
i) D-Glucuronsäure oder UDP-D-Glucuronsäure, und
ii) N-Acetyl-D-Glucosamin oder UDP-N-Acetyl-D-Glucosamin, in einer kosmetischen Zusammensetzung einkapseln.

9. Verwendung eines Präparats gemäß den Ansprüchen 1 bis 7 zur nichttherapeutischen kosmetischen Behandlung von Falten, Hautfalten, Aknenarben, posttraumatischen Narben, Rhinoplastik-Narben, gelifteten Lippen, Wangen oder Kinn.

## Revendications

1. Préparation cosmétique contenant des vésicules de type liposome chargées avec i) de l'acide D-glucuronique ou de l'acide UDP-D-glucuronique, et ii) de la N-acétyl-D-glucosamine ou de l'UDP-N-acétyl-D-glucosamine, dans une composition cosmétique.

2. Préparation selon la revendication 1, contenant en outre un inhibiteur de hyaluronidase.

3. Préparation selon la revendication 2, dans laquelle ledit inhibiteur de hyaluronidase est un extrait d'*echinacea angustifolia.*

4. Préparation selon les revendications 1 à 3, dans laquelle les vésicules sont constituées essentiellement de phosphatidylcholine.

5. Préparation selon les revendications 1 à 4, qui est sous la forme d'une crème, d'une émulsion, d'une microémulsion, d'une pommade, d'un gel, d'une lotion ou d'une huile.

6. Préparation selon la revendication 5, qui est sous la forme d'un gel contenant de l'eau et du glycérol.

7. Préparation selon la revendication 6, contenant en outre de l'EDTA, du pyroglutamate de sodium, du lactate de sodium et un agent épaississant, de préférence de la gomme xanthane.

8. Utilisation de liposomes encapsulant
i) de l'acide D-glucuronique ou de l'acide UDP-D-glucuronique, et
ii) de la N-acétyl-D-glucosamine ou de l'UDP-N-acétyl-D-glucosamine, dans une composition cosmétique.

9. Utilisation d'une préparation selon les revendications 1 à 7, pour le traitement non-thérapeutique cosmétique de rides, de plis cutanés, de cicatrices d'acné, de cicatrices post-traumatiques, de cicatrices suite à une rhinoplastie et de lèvres, joues ou menton remodelés.
